Europäisches Patentamt

European Patent Office  (11) Publication number: **0 347 526**

Office européen des brevets  **A1**

(12) **EUROPEAN PATENT APPLICATION**

(21) Application number: 89104884.5

(51) Int. Cl.4: **A61K 31/00**

(22) Date of filing: 18.03.89

(43) Date of publication of application:
27.12.89 Bulletin 89/52

(84) Designated Contracting States:
BE CH DE ES FR GB IT LI NL SE

(71) Applicant: Zagyansky, Y.
chez Mme Kelepovsky 28 rue de Tolbiac
F-75013 Paris(FR)

(72) Inventor: Zagyansky, Y.
chez Mme Kelepovsky 28 rue de Tolbiac
F-75013 Paris(FR)

(54) The fundamental structure of the action of the hormons, the neurotransmetters and the growth factors in the transmission of a signal in the cell and its very important consequences.

(57) The invention includes the new knowledges permitting to create the principally new structure of the action of the hormons, the neurotransmetters and the growth factors. The main, principally new components of this structure are the following argumentations. The creation of the complexes: ligand → receptor → lipids according to the plan: receptor → ganglioside → cerebroside → → sphingomyelin with the help of the intercarbohydrate and interlipid hydrogen bonds. The modulation of the structure of the interior membrane layer to create the association sites with the mobile proteins as the protein kinase C or the regulatory G proteins with the help of very long fatty chains of the exterior layer. The postulation of the role of the endocytosis as the means of the negative feedback regulation of the enzymes of the interior membrane layer, in particular the protein kinase C. The postulation of the micellar rotational structure of the $Ca^{2+}$ transfer through the plasma membrane with the help of the lysophosphatidic acid after the action of the phospholipase $A_2$ and acyl-transferase. The new preparations are proposed against ten or so mental diseases- sphingolipidoses, against the cancer and for the anaesthesia execution as the important practical consequences of this structure. It is proposed the creation of the Universal Maps of the action of the particular hormons, the neurotransmetters and the growth factors according to the fundamental structure with the corresponding new preparations for the recovery, which approach the state of the action of the receptors in the pathology state to their action in the normal one. The new, apparently very perspective, preparations, containing the antibodies with the modified charges, with the very different way of their action, are proposed against the viruses (the HIV virus included) and the bacteries.

# THE FUNDAMENTAL STRUCTURE OF THE ACTION OF THE HORMONS, THE NEUROTRANSMETTERS AND THE GROWTH FACTORS IN THE TRANSMISSION OF A SIGNAL IN THE CELL AND ITS VERY IMPORTANT CONSEQUENCES

The communication plays an important role in the life of a cell of an organism which have milliards of such cells. To attain this object, the special and selective molecules, which are situated, in general, on the membrane surface (I do not examine here the steroid and other hormons with the similar action), interact with the communication messengers (ligands): the hormons,the neurotransmetters and the growth factors; these ligands switch the sequence of events (signal) that induces the adequate to the state of an organism cellular answer (the mitosis, the synthesis of molecules, the differenciation, the exocytosis).

Being the key subject of numerous biological and medical disciplines (endocrinology, pharmacology, immunology, physiology, neurology, toxicology, pathology), this question takes attention of very numerous laboratories of the basic and applicative tendency of Universities, Institutes and Hospitals of all the world. In spite of the recent spectacular explosion of new knowledges (where the informations are becoming old only in two years) in the established structure and the action mechanism (and in the based on them generalisation) of the receptors, the G proteins, the second with phosphatidyl phosphoinositol (PPI) and the protein kinases, there is no yet a profound knowledge of events taking place from the membrane receptors and in consequence, the reason of the presence of the numerous components in the cell membrane rests a true mystery today.

The numerous points, concerning the transformation of the signal through the membrane, rest to develop. Fortunately the numerous divergent scientific results concerning this key domain of the life sciences and a series of my own key works in the domain of the begining of the signal in the cell and the comprehension of the signal action permitted me to create and to develop the fundamental and original structure of an action of the hormons, the metters and the growth factors. The derangements during the actions of these physiologically important substances must lead (and unfortunately have led) to the numerous existing diseases. As the consequence of this fundamental structure, I present here the very important and urgent practical developments: the preparations against ten or so diseases of new-borns, against the cancer and for the execution of the anaesthesia and against the viruses.

My original and fundamental structure is exposed below. After an interaction of the ligand with the receptor there is a conformational change of the receptor (1,2). It must be sufficiently fort to permit, according to my proposed structure, the carbohydrate chains of the receptor-glycoprotein to have the interaction with the carbohydrates of the glycolipids (3). The fact of the presence of strong similitudes of the terminal carbohydrate structures of the membrane glycoproteins with the carbohydrate structure of the glycolipids (4,5) can permit their stronger interaction with the hydroxyl groups of their "chair"-structures which must have the corresponding superposition. In the systems of the carbohydrates having the strong hydrogen (H) bonds, the solvent molecules (water), in general, are excluded during this interaction (6,7). It is necessary to consider the differences between the parts of the proximal carbohydrates of the glycoproteins and the carbohydrates of the glycolipids (4).

I suggest that the ganglioside molecules are the first of all glycolipids (or rather of the glycosphingolipids) which react with the receptor-glycoproteins, because on one side there is the specific interactions and the electrostatic attraction of the negative charges of the gangliosides which can react with the negative charges of the proteins by means of the $Ca^{2+}$ bridges (8) /The degree of the $Ca^{2+}$ association with the gangliosides is similar to that of the negative phospholipids (9), permitting to the molecules-"chairs" to approach and to have stronger interactions/. On the other side the greatest hydration and the greatest dimension of the head group must amplify a signal effect making from the ganglioside a first more sure reactive /the quantity of the freezed water for the gangliosides is much more important than that for the cerebrosides (10)/. The clear fact that, from the gangliosides, there is already the formation of the patches and the caps with the antibodies against the dye groups conjugated with these gangliosides and evidently situated uniquely on the gangliosides of the T lymphocytes (11), argues also for the priority of the gangliosides. The participation of the gangliosides (the antigens I and i) in the process of capping, without the creation of direct links with the ligand Concanavalin A (Con A) points out the same conclusion (12). The fact of primary action of the toxins, the viruses and apparently the other agents became ambigous after the numerous experiments (13,14) which indicate the glycoproteins as the veritable virus and toxin receptors, that is closer to my proposed plan, see below).

Thus one can explain already the experiments of Kanfer et al (15) which were not understandable easily until now. With the weak concentrations of the cholera toxin there was no effect of the toxin because these concentrations were not sufficient to react with the corresponding glycoproteins (molar concentration weaker than that of the corresponding gangliosides). On the other hand with very strong toxin concentrations, there was apparently a blocking of the necessary groups of all gangliosides by the relatively large toxin molecules. This new explanantion interprets easily the suppresion of the signal with the excessive quantities of Con A. This fact is the subject of the numerous discussions since longtime (16). The experiments with the multivalent 1ibands, reacting only against the gangliosides, show an absence of patches and confirm this interpretation (14,17). Morever the experiments concerning a positive action of the monovalent Con A and Ricin (RCA 60) (18, 1 9) are explain nable in considering the change of conformation of the protein-receptor (like the action of the monovalent hormon).

The experiments concerning the mechanism of the action of the pertussis toxin confirm this explanation. According to these experiments (20), the hydrolysis of the sialic acid and the groups of N-acetylglucosamine ($3^{TH}$ from an extremity of the carbohydrate chain of receptor-glycoprotein) diminishes strongly an affinity of the carbohydrate chain during the interaction with the toxin. But the hydrolysis of the glucose ($2^{nd}$ from the extremity) do not change this affinity. Morever due to the synthesis of this carbohydrate chain without this glucose, the mentioned affinity augmented even. Knowing, for instance, that the optimal quantity of the carbohydrates in the active site of antibodies is approximatively near 5 one can suppose that only these groups (sialic acid and N-acetylglucosamine) interact with the active site of the toxin in approaching and in provoking a stronger tension of the chain because the intermediate groups of the glucose apparently do not make this interaction.

Now I would like to show why the membrane glycosphingolipids have the particular properties which permit them to be the lipid initiators of the signal in the membrane. The observation of a behavior of the natural sphingolipids during the transition temperature shows that there is the presence of a metastable state. This metastable state is characterized by the participation of the H interlipid bonds (21-23).

A more stable phase is characterized by the hydration of the lipid surface groups and by the rupture of the intermolecular H bonds (24). The sphingolipids have groups in their backbone which are donors and acceptors of the H bonds. The orientation of these H bonds of the donors and the acceptors (the amide groups and the hydroxyl groups) permits these molecules the lateral interaction with the other lipid molecules (21). If these OH groups are exposed to the water, coating the membrane lipids, the individual H bonds between the molecules (lipid-$H_2O$) do not need to be strong to reclose (lipid-lipid) from one molecule to another (25). The lateral segregations of the sphingolipids forming the patches are well known (26). But this "magic" metastable state is not only the property of the sphingolipids, because even the phosphatidylcholine (PC) can have the metastable phase similar to that of the GLYCOSPHINGOLIPIDS after a transformation of its acyl chain into "acyl-carba moyl" one that manifests like the H bond donnor via the NH group (27). That is , the metastable phase of the sphingolipids is characterized by the H bond between the molecules but not by a specific conformation or by a steric configuration of the head groups or of the ceramic backbone.

...In the proposed fundamental structure, I suppose that, after this strong dehydration produced because of stronger interactions of the ganglioside carbohydrates with the glycoprotein ones, there is a transition of the lipid ganglioside molecules in a state characterized by the H bonds between the chains after the conformational change and very probably the dehydration provoked by the interactions indicated above. The fact of the total inhibition of the thrombocyte aggregation, only after the replacement of the water by $D_2O$ without the change of the ligand association (28), preaches in favor of the mentioned importance of the water in the signal transmission through the membrane, although I cannot determine, according to the proposed structure, the exit of all similar experiments. An other important fact (and not accidental) is very interesting. The regions of the receptors, like the muscarinic receptor (29), $\beta$-adrenergic receptor (30), or the low density lipoprotein receptor (31) /that participates also in the signal transmission- (32)/, locating near the places of a carbohydrate fixation and having the interaction with the glycosphingolipids, have a small quantity of the charges. This must help to the mentioned profound dehydration and indicates the correctness of the proposed structure. The results obtained with the paramagnetic probe indicate the redistribution of the H bonds of the ceramides and the cerebrosides after the interaction of the ligands (Con A and phytohemagglutinin) with theirs receptors (33).

The fact, that the gangliosides (contained the intact ganglioside carbohydrate chain with the degenerated lipid chain) cocap with the caps (contained the membrane immunoglobulins with theirs antibodies) on the lymphocytes (34), confirms the order of the events indicated above. The results of the nuclear magnetic resonance (NMR) (35) show the dependence between the conformation of the acyl chain and the carbohydrate head group in the glycolipids. One finds that the same dependence between the conformation of the head group and the lipid chain in the experiments with the synthetic and natural molecules of sphingomyelin (SM) with the different length of the chains. Only the molecules of stearoyl-SM had the transition with the metastable state (23). The dependence of the cerebroside accessibility to theirs antibodies on the lipid matrix (36) shows also the validity of the above interpretation.

One can suppose that, after this interaction of the carbohydrates with the mentioned dehydration, the lipid parts of the gangliosides can easier have the interactions which correspond to the metastable state with the intermole cular H bonds. This"cristallisation", from a protein center (receptor) after the interactions of the gangliosides, that have a similar structural plan of the carbohydrates, must continue with the cerebrosides. Again one can see here a confirmation. The majority of the cerebrosides has the glucose and the galactose in the head group. One finds them, which are free from the interaction with the glycoproteins, in the proximal part of the gangliosides, that is the another confirmation. I propose that the cerebrosides constitute the part of this metastable "cristal" (patch) in the second "concentric circle" around the receptor with the carbohydrate interactions and, more importantly, with the interlipid H bonds. Apparently, from some dimension (that reflects a larger changes accompanying the withdrawal of the water from the corresponding part of the membrane), the lipid interactions are sufficient because the lipids like the ceramide (without the carbohydrates) are agglomerated in the patch (33,37,38) (there is only the weak quantity of the ceramide in the natural membrane, reflecting the fact that it is the metabolism product).

The SM molecule with the length of the acyl chain 18C (18 carbon atoms) (18:0), that has a mentioned metastable phase and characterized by the intermolecular H bonds (23), can conceivably participate by its lipid part in this growing patch by the interlipid H bonds. But the other natural SM molecules cannot, apparently, participate here (39). The results of NMR indicate the presence of the intramolecular H bonds in these molecules (40). Very probably this reflects the absence of the metastable state of the SM natural molecules (23) and the constancy of the specimen of the X ray diffraction of the SM bilayers exposed to the different humidities (41). And seemingly, to "attract" all the SM membrane molecules the Nature found an original solution.

One knows that the SM of the mammalian membranes have the transition temperature about 37° C (42). One knows equally that more than 50% of the plasma membrane SM have more than 20 carbons in theirs acyl chains (43). And morever the packing of the SM lipid chains in the bilayer is very dense and there is no any interdigitation of the adjacent molecules in the same bilayer (41). That is there is a strong dissipation of the free energy during the phase transition of SM. And when with the intramolecular H bonds, the SM molecules (which have the chain length 18:0 and the transition temperature evidently more than 37° C) are taken in the mentioned patch, the rest of the other SM molecules must have the gel state without complementary influences about the same "cellular" 37° C. It is probable that the ordered mosaic of the SM molecules with the very long and less long chains must arise in the patch around the receptor. The mosaic of the fatty chains of this phospholipid having the described "concentric circle" can appear and around dimers of the epidermal growth factor (EGF) and insulin receptors (44) having smaller dimensions, which (dimers) are formed after the action of the ligand without a negation of the independent conformational change (45).

But after this creation of the SM domains (even as the "exterior ring"), one can suppose naturally that there is an interaction of the cholesterol with SM (25,46) because the preferential interaction of the cholesterol with SM can happen only with the domains of SM but not with the individual SM molecules (47). I suppose that this redistribution of the cholesterol in the membrane plane must lead to the appreciable diminution of the quantity of the cholesterol in the rest of the membrane that must provoke the phase separation (48) after the interaction of the ligand with the receptor (49). In consequence one can suppose the creation of the fluid zones with the phospholipids which have a weak affinity for the cholesterol or because of theirs lipid chains or because of theirs heads. For example, the phosphatidylethanolamine (PE) has a such affinity to the cholesterol (50) and must be apparently in this lipid zone. The publication of Broekman et al (51), according to which the phospholipase $A_2$ acts preferentially on PE during the activation of the thrombocytes, argues for this. The fact of the presence in the exterior layer of only 10% of the arachidonic acid (AA) of the total quantity of AA in the membrane (52) and the fact of the presence in the exterior layer of at least 50% of AA of the total quantity of AA in PE (52) /giving a stronger garanty of the action of the phase separation, registed by Zagyansky and Jard (49)/confirms the mentioned propagation. The PC molecules, with the cis chains and well unsaturated as AA (20:4), must have a weak affinity to the cholesterol because, in the case of the bilayer of PC (46), the cholesterol extends only until the twelfth C atom (46). It is evident that the different elbows of AA are an obstacle to the best interactions with the cholesterol. The experiments with the polyene antibiotics,which make the specific interactions with the cholesterol and even according to Shroeder (53) eliminate it from the membrane by making the complex, confirm also the validity of the proposed structure with the participation of the cholesterol. According to Zagyansky and Jard (19),the amphotericin B eliminates the biological and physical effects of the ligand action and according to Williams and Lefkowitz (54) the filipin molecule makes an obstacle to the association of the agonist with the β-adrenergic receptor. The presence of the active site of the receptor of this type near the biological membrane (55,56) facilitated apparently the obtaining of these results. According to mcconnel and coworkers (57) the cholesterol accumulates near the interface: liquid-solid to diminish the surface tension. This conclusion of McConnel and coworkers coincides with my suggestion that just the SM molecules, which have the upper affinity to the cholesterol and which have no intermolecular H bonds, must be near the frontier: liquid-solid.

The conclusion that the SM has an intramolecular bond between the 3hydroxyl group and the oxygen of the ester part (40) and the conclusion that the cholesterol makes the interaction with the carbonyl of the amide group of the sphingolipids by the H bond (58,59), confirm the fact of a stronger interaction of the cholesterol with SM. On the other hand, very probably, the NH groups of the glycosphingolipids participate in the intermolecular interactions of the lipids and their interaction with the cholesterol must be weaker. The interpretation of the experimental confirmation of this creation of more fluid zones around the patch with the receptor is concluable from the experiments of Curtain et al (33) (The PC with a modified chain could not have the strong interaction with the cholesterol and its microsurrounding became more fluid after the signal).

Huganir and Greengard (60) classified the membrane receptors in 3 groups: 1)the receptors which activate effectors by the G proteins, 2) the receptors which are the tyrosine kinases, 3) the receptors which make the conductibility of the ions by theirs conformational changes. /According to (61), the metabolites of AA do not function as the circulating hormons but are rather the parahormons or autocoids and make their action at the level of the second messengers. See also (62,63)/. The receptors of the 3rd group (like the nicotinic acetylcholine receptor) are not the subject of this invention although the essential part of the conclusions of the proposed structure is applicable for the action of these receptors/. On the other hand one can divide the first group in two subgroups: the receptors which activate the phosphatidylinositol cycle and the receptors which activate or inhibit the adenylate cyclase. In addition to this the G proteins must apparently participate in the action of the receptors of the second group (64).

In reality the classification is much more complicated because there is the presence of the homogeneous and heterogeneous interactions of the receptors (60,65-67) or for instance, there is the presence of the different forms of the isoreceptors with the different action mechanisms (68 and for instance 69). In consequence the signal is determined as a sum after a superposition of the components of the cycles and the knots interferenting between them that depends on a cellular type, a tissue type, a cell state in a particular moment, concentrations of different substances, a presence of other signals. But nevertheless one can reveal the essential knots of the signal and even common for the mentioned above cases after all numerous manifestations of the cellular activity observed often in the cellular cultures being in the conditions which are different from ones of an organism, in the established lines and theirs mutants having the modified quantity of chromosoms, or in the cultures of the cancer or transformed cells.

I think that these common knots include the complexes of the ligands with the receptors, the glycolipids and the phospholipids having a high affinity (see above) and being longtime on the cell surface during the signal. These common knots include also the complexes of the ligands with the receptors and the lipids having a weak affinity and, as I propose for the first time, serving for the regulation of the enzyme activity on the interior membrane layer. The presence of these receptors of the different affinity was observed in all the receptor types (and as I suppose convincingly, it can be revealed in all receptors):the receptors with the PPI cycle, with the cAMP action and with the tyrosine kinase action (see below).

These complexes with a weak affinity (that is not itself constant and, apparently, diminishes with the propagation of the signal- see below) do not locate during longtime on the cell surface. They are endocyted through the coated pits. The products of this endocytosis serve only for the regulation of the action of the affined but less numerous complexes in all the considered receptor types, resting during longtime on the cellular surface (see below). The serious confirmation of the generality of the processes, taking place on the cell surface, is the presence of the sustained $Ca^{2+}$ current through the plasma membrane in all receptor types (70-72). Later the common knot of all considered receptors must be the protein kinase C (see below). The mentioned above endocytosis synchronized with the signal current must serve for its regulation (at least) like the feedback. As I think (see below), the regulation of the "adressable" association of the peripheric and temporary proteins like the protein kinase C or the G proteins /also presenting in all signals -(64)/ takes place for ALL signals: 1)by the mosaic of the long fatty acids of the exterior membrane layer during the creation of the mentioned complex of the sphingolipids with the receptors which must be individual for each signal, 2)by the system of the well regulated and synchronized inclusion of the unsaturated fatty acids in the phospholipids of the interior layer as the consequence of the augmentation of the transformation of the lipid exchange in the cell with the signal propagation.

In the large quantity of the receptors of all considered types (73), with the propagation of the signal,there is an increase of a pH in the cytoplasm that is the consequence of the action of the $Na^+$/$H^+$ exchanger activated by the protein kinase C (see below). As a result of the signal, the sodium enters in the cell and there is an activation of the synthesis of RNA, the protooncogenes and DNA in the case of the mitosis (74).

As I think too receptor types with the very different affinities are just a result of the mentioned above interactions (75-80). The following arguments give an evidence in favor of this. Harden et al (81) separated in the density gradient the $\beta$-adrenergic receptors before the interaction with the ligand obtaining the continious spectrum of the receptors with two weakly ex-pressed maximums in the density diapason. After the interaction with the ligand, on the cell of course, there is a strong redistribution of the number of the receptors, and two picks become expressed clearly. An essentially larger pick corresponds to the receptors with a less strong activity. This is for the first time that I propose that there is a dynamic equilibre between these two receptor forms. Apparently, the components like the glycolipids, with which there is the creation of the ligand-receptor complex, change the receptor affinity to the ligand and they are just such migrating components between the different receptors, which make the mentioned dynamic receptor equilibrium, that is displaced in the presence of the ligand, morever in the stable forms. According to Hakomori and coworkers, for instance, the $GM_1$ and $GM_2$ gangliosides increase the affinity of the receptors for the growth factor (2). Or, for instance, according to the proposition of Chorev et al (82), the derivative of the irreversible antagonist binds to the active site of the receptor while its bromacyl tail makes the interaction with the lipid molecule that participates apparently in the creation of the active site. This new hypothesis could explain clearly the mystery of 12 years of an absence of the complete recovery of fluorescence of the complexes of the receptors (83,84) with or without the ligands in the native cellular membrane, begining with the first in the world, in spring 1975, experiment without the ligand in the active site (85) by the fluorescence photobleaching recovery (FPR). Morever in spite of the presence of the zones with the restricted mobility after the interaction of the receptor with the ligand (49), that influences remarkably the percentage of the fluorescence recovery, there is the presence of the "immovable receptors" because the percentage of the fluorescence recovery in this experiment increases from 30 to 50 after the repeated photobleachings although there is no 100% of the recovery because of the zones with the restricted mobility (all over the cell) of about 100% of the free receptors, without the ligand, in the electric field (19,86-88) is well resoluble, and, apparently, essentially in accordance with the introduced hypothesis, even with an apparent contradiction with the presence of a large persentage of the "immovable receptors" /There was no any mobility of the receptors in all these experiments in a certain percentage of cells, probably, for instance, because of the presence of the cells in the different stages during the cell cycle- (89)/.

I think that two large receptors subgroups, revealed during the interaction of $\beta$-adrenergic receptors with the lectins conjugated with the agarose (90), must reflect just the presence of the conformational change in the receptors which are seemingly in the complex with the most close sphingolipids. This must provoke a different receptor accessibity to the lectins on the agarose and an appearance of two types of the affinity for the receptors without the ligands. One can suppose also that, because the $\beta$-adrenergic receptors are the receptors for Con A, there is a displacement of an equilibrium between two receptor types without the ligands after the interaction with Con A (81) since apparently there is a change of the sphingolipid microsurrounding.

According to De Meyts et al (91) the occupation of the active site of the insulin receptors by the ligand molecules begins from the more affined receptors continuing with the less affined ones. As I suppose, the constituentes, like the gangliosides, for the strong affinity state, being in the limited quantity in the cell membrane,are "teared out" by the receptors reacting first, in addition to this, apparently, statistically. The fact, that the Con A receptors, which are more numerous than the insulin receptors, take in theirs active sites these essential but deficient components, indicates also for this suggestion, because, after this intervention of Con A, the Scatchard plot become linear reflecting only the presence of the weak affined active sites. Also the Scatchard plot became linear,with the weak affined active sites, after the analogous modulation of the membrane immunoglobulins G by the Con A receptors (91). The fact, that for an elimination of the most affined active sites of the different receptors, the interaction only with Con A would be necessary, proves clearly that seemingly this must concern the molecules having the corresponding carbohydrates. It is very probable that this concerns the gangliosides, confirming clearly the proposed structure. Apparently the numerous interactions of the different lectins block the association of EGF with its receptors ($37°C$ and $4°C$) (92). In considering all these experimental results, one must see that, in reality, the active sites of the receptors must have a stronger affinity during an association with the antagonists than that obtained already after manipulations with them (93).

After the experiments of Pratt and Pastan (94) it is possible to see that the affinity of the active site is created with the assistance of the carbohydrate interactions and in consequence, after my proposed structure, with the glycolipid assistance, because the mentioned analogous change of the insulin receptor affinity takes place apparently in this case when the EGF association with the receptors of the mutant cells deprived of the N-acetylglucosamine synthesis is only a third of the association of the normal cells ($37°C$ and $4°C$) and is only 13% with the receptors of the mutant cells transformed with the tunicamycin that inhibits the carbohydrate association with the receptors. And once more seemingly, the same case of the "tearing away" of the "deficient" components with the diminution of the association constant takes place after the irreverisble association of the $\beta$-adrenergic an tagonist ("affinity label") that has a covalent bond with the lipid that constitutes the active site. With this the mentioned antagonist can be associated only with the half of the active sites (82). The fact that the receptor oligomer structure is not known, confirms this conclusion (95). I think that there are two types of events during and after the creation of the complexes, mentioned above, for all signal types. Firstly, there are the events which take place in the exterior membrane layer which lead on one hand to the increase of the $Ca^{2+}$ current in the cell through the plasma membrane and on the other hand to the desensibilisation and the endocytosis of the receptors with the ligands. And secondly, there are the events propagating apparently because of the conformational change of the receptor in the interior with the activation of the tyrosine kinase, the adenylate cyclase, the protein kinase C. Besides this there is also the modulation, as I propose for the first time, of the redistribution and the conformation of the fatty glycolipid and phospholipid chains of the interior membrane layer with mainly the "intentionally" long sphingolipid chains of the described above complexes that leads to the change of the localisation of the different proteins like the G proteins,the protein kinases and the phospholipases and their substrats between the interior membrane surface and the cytoplasm and,as I think, that determines the localisation,sufficiently precisely, of these peripheric proteins in the niches prepared for them in the membrane.The niches must be sufficiently well determined for each receptor type because niches are determined by the plan of the interaction proposed above:receptor → ganglioside → cerebroside → sphingomyelin evidently individual for each receptor.

Let us see the events taking place in the membrane exterior layer. Very probably, the redistribution of the lipids on the membrane surface and the creation of the different phases after the ligand action leads to the acti vation of the phospholipases C and D which are specific for the glycosylphosphatidylinositol molecules containing the glycan, the ethanolamine and the protein interconnected by the covalent bonds with the phosphatidylinositol (PI) (96,97).Such large expansion of these molecules in the plasma membrane of the different cells (98) confirms the general character of the action of the phospholipases C and D in this case during the different signals. Such hydrolysis leads to the appearance of the diacylglycerol (DG) molecules (99-101). Apparently, the phosphatidic acid (PA) must appear immediately (102) or after the action of the phospholipase D in the exterior membrane layer (96) or after the delay due to the action of the diacylglycerol kinase on DG (103,104). In addition to this, the activity of the specific phospholipase C for PC (its action was revealed in the exterior membrane layer in some seconds) contributes to the appearance of DG. This fact of the presence of the small pick, that is close to the signal begining, shows that the ac tion of these phospholipases of the exterior layer takes place (105):this pick has the relative dimensions which are similar to these in the experiment of Griendling et al (106) after the action of the pertussis toxin that inhibits the G proteins situating in the exterior membrane layer and participating in the action of the phospholipases C (also $A_2$ and D) of the membrane interior layer (107) (see below). Apparently with this, the favorable reseau of the H intramolecular bonds in the phospholipase $A_2$ molecule, that is necessary for its activation (108), is created, considering the creation of the mentioned intermolecular H bonds of the sphingolipids and the dehydration near the exterior lipid structure. The fact of the diminution of the phospholipase $A_2$ activity with the action of the anaesthetics (109) confirms this argument. It is proposed that the anaesthetics suppress the H bonds in their action (25,110). That is after my proposed above structure,one can deduce a concrete and practical mechanism of the anaesthesia:the suppression of the hydrogen bonds between the sphingolipid molecules.The experiments of Hirata et al (111) prove the presence of the activation of the phospholipase $A_2$. The antibodies against the lipomodulin molecules (phospholipase $A_2$ inhibitor) applied outside of the cell, increase the activity of this enzyme approximately in 3 times without the hormon and in 12 times with the hormon.So the action of the different enzymes on the cell surface leads to the temporary formation of DG and the creation of PA almost from the begining of the signal (112)-.Apparently,in all receptor types, the PA serves for the $Ca^{2+}$ current through the membrane by a mysterious mechanism yet:an another proposed mechanism (with the PPI metabolism) has no essential confirmations (113).

Afterwards, there is an activation of the protein kinase C in all cases. In the case of the "PPI"-mechanism, this activation takes place because of the temporary augmentation of $Ca^{2+}$ in the cytoplasm and the increase of DG (114) and in the other cases, apparently because of a direct DG action (97, (101). There is the numerous results which indicate that the protein kinase C participates in the signal action in the systems without the PPI cycle (71,101,115-124). And morever one knows that the protein kinase C influences the $Na^{+}/H^{+}$ exchanger and the hydrogen ions leave the cell in increasing the pH in the cytoplasm (73,74,125-128). This increase of the pH was registed in a very large number of the signals of all types (73), confirming respectably the fact of the activation of the protein kinase C in all these signals.

The DG, in the interior membrane layer, apparently is formed from several sources. It is the specific for PI phospholipase C (129,130) with the activity depending on the $Ca^{2+}$ current through the membrane (103,131). In addition to this there is the specific for PC phosphatidylethanolamine (PE) / phospholipase C that has apparently its G protein (130,132-134). This G protein can be activated by the protein kinase C (134). The specific for PC(PE) phospho'lipase C do not need the $Ca^{2+}$ current through the membrane (131) like the phospholipase D of the interior layer for PC(PE) that has also its specific G protein (135,136). The activity of this phospholipase D can be increased by the protein kinase C (136).

The kinetic of the DG formation has often two picks (102,103,137,138). The precision of the method is not very great because DG was mesured in the membrane and in the cytoplasm. And for instance, in the case of the adipose cells, an increase of DG, due to the PI degradation,is very difficult to see since the DG quantity in this cell type is very important (139). I think that the first pick corresponds to two described above reactions:the DG formation in the exterior membrane layer due to the hydrolysis of the glycosylinositols and PC (PE) and in the interior membrane layer due to the PPI hydrolysis (106,138,140). With the activation of the protein kinase C,there is an inhibition of the phospholipase C activity hydrolysing specifically PPI (106,123, 130).Apparently,the second pick must be present due to the protein kinase C action because one can stimulate it with the phorbol ester (137,138).Probably this pick is formed due to the activation of the phospholipases which hydrolyse PC(PE) (106,130,133,135,137) and/or PI (106,129,130) with the stage of the G protein phosphorilation by the already activated protein kinase C molecules. After or/and during the activation of the phospholipases C,there is apparently the activation of the phospholipases $A_2$ of the interior layer (112). As it was mentioned, the phospholipase $A_2$ activation is favored with the phase separation by the phospholipids with the negative charge and by the $Ca^{2+}$ ions. In addition to this, the products like the DG, which are the results of the signal, favor the displacemant of the equilibrium of the presence of the phospholipases $A_2$ between the cytoplasm and the interior layer in the direction of their presence in the membrane (141). In addition one must mention the presence of the numerous forms of the phospholipases $A_2$ - (see, par example (142)/.The protein kinase C favors also the activation of the phospholipase $A_2$ by the inactivation of its inhibitors (lipomodulin and lipocortin) (143,144).The activation of the phospholipase $A_2$ with the phorbol ester manifestates also in favor of this (145). The charged negatively phospholipids (PA) (which activate the phospholipase $A_2$) (146) and the $Ca^{2+}$ ions present themselfs as the action of the $Ca^{2+}$ current through the membrane, as the action of the phospholipases D activated by the protein kinase C with the G protein in the interior membrane layer (136) or as the action of the diacylglycerol kinase on DG (131)-.As I think,the same components favor a certain localised separation of the phospholipids in the interior membarne layer with the creation of the vacant zones for the peripheric proteins (the protein kinase C and the phospholipase $A_2$ included) (see below).

With time the DG quantity diminishes in favor of the activation of the diacylglycerollipase that depends on $Ca^{2+}$ (147,148) or in favor of the diacylglycerol kinase action (149) increasing the PA accumulation (102,112,150).

Very probably the diacylglycerol kinase hydrolyses the fatty chains in the C2 position only (147) and in consequence the monoacylglycerol (MAG), well migrating from the bilayer, can be found in the sufficient quantities outside, in the solution, and inside of the cell (148). This metabolism chain must lead later to the PI cellular synthesis by the lysophosphatidic acid and PA (without DG as the intermediate product), with a probable A inclusion /see below the details of the correlation with the works of Resch and coworkers (151,152)-/.This inclusion do not proceed after the phospholipid synthesis in the cell de novo (153)-.The fatty acids (AA included) are incorporated in the phospholipids or from the endocyted lipoproteins (154) or from the cellular reservation (155). With the action of the signal, the quantity of the free fatty acids must increase (51) creating the dynamic current of these fatty acids in the membrane direction (156) although the level of the fatty acids must be very weak due to the process of the esterification of the fatty acids with their inclusion in the phospholipids (157). The triacylglycerol (TG)-,the fatty acid stock,is hydrolysed, in general, by the lipases situating in the lysosomes or in the plasma membrane (158-160). /The activation of the lipases located in the plasma membrane serves,apparently,for the hydrolysis of TG, accumulated in the plasma membrane with the signal activation,this accumulation can be executed with the phorbol ester (161)/.The phospholipid synthesis do novo (162) during the signal was registed in the curtain number of cases from the glycerophosphate formed after the glycolysis (102).Apparently these phospholipids are included in the cell membrane with the system (activated by the signal) of the vesicular membrane of Golgi and endoplasmic reticulum (138, 163), for instance with the mechanism including the G protein (164) or/and, as I think,with the activation of the protein kinase C,that increases an efficacity of this transport process directed from the intracellular Golgi and endoplasmic reticulum membranes to the plasma membrane in profit of the phosphorilation of the assembly proteins included in the regulation of the structure and the function of the coated pits (165). It is evident that the more the endocytosis is intensive,the more the membrane lipids go to the lysosomes.

This formulation helps to clarify the role that I have proposed for the protein kinase C in the creation of the coated pits with the phosphorilation of theirs assembly proteins. According to the experiments of Larkin et al (166), the quantity of the coated pits and the degree of the endocytosis diminishes with an exposition of the cells in the buffer without $K^+$. After the experiments of Ramussen and coworkers (167), one knows that there is an in crease of the sustained $Ca^{2+}$ current in the cell with an increase of the $K^+$ ion concentration in the exterior buffer (with the correspondent hyperpolarisation). That is in the case of the experiment of Larkin et al,there is a diminution of the basal level of the $Ca^{2+}$ current in the cell through the membrane (do not confound with the voltage dependent channels) (see above). As I think, this must lead to a diminution of the basal level of the protein kinase C activity depending on the $Ca^{2+}$ concentration (114),that is a diminution of the protein phosphorilation of the mentioned assembly proteins (169).One can see the confirmation of the structure of the endocytosis regulation with the coated pits in the experiments of Kolesnick (169) according to which the DG stimulates an increase of the SM hydrolysis in the whole cell. One can explain these experiments with an increase of the protein kinase C activity after which a certain number of the coated pits and the degree of the endocytosis increase. In consequence a stronger quantity of SM goes in the lysosomes where it is hydrolysed.

Finally, one can see (on one hand) the processes of the degradation and the elimination of the lipids of the plasma membrane and (on the other hand) the synthesis and the incorporation of the lipids in the plasma membrane, which are logically ≈ equal in mass. Naturally one can ask the question to know why the is a necessity for this dynamic lipid current. It is possible to see a systematic increase of the inclusion of the unsaturated fatty acids and the inclusion of AA in the phospholipids after the experiments of Resch and coworkers with the stimulation of the mitosis of the T lymphocytes by Con A (170-172). These and the other authors demonstrated that for instance the activity of the $Na^+/K^+$-ATPase or the lysolecithin acyltransferase increases but the activity of other enzymes (for instance the phosphatase) diminishes (173,174).With this,one can simulate the same effect of the activity modulation of the mentioned enzymes by the simple addition of unsaturated fatty acids (in the restricted limits) to the membrane fragments of the lymphocytes (175,176). Morever the effect of the modulation of the membrane enzymes by the degree of the unsaturated fatty acids included in the lipids has a larger expansion (178) increasing, for instance, the activity of such enzymes as the adenylate cyclase. According to Rozengurt (128) or Berridge (74), just the enzymes, which change the ion concentration of $Ca^{2+}$ and $Na^+$ (that is, for instance, the mentioned $Na^+/K^+$-ATPase) are associated by the necessary means with the development of the signal and with the induction of the DNA synthesis and the cell growing, and, for instance, an inhibition of this enzyme leads to the arrest of the mitosis in the first stage (174,176). Morever, according to Li et al (178), the activity of an other ATPase ($Ca^{2+}$-ATPase) must have a direct dependence on the degree of the fatty acid unsaturation of the bilayer because the composition of the lipids surrounding this ATPase depends directly on the total concentration of the unsaturated membrane lipids (179). At last, one can see that these knot-works (describing the metabolic pathways of the well synchronized events nevertheless, and taking place during the signal) are necessary for the modulation of the degree of the lipid saturation in the plasma membrane, that (the degree) changes with the signal propagation and do not change even after a simple exposition of the intact lymphocytes with the fatty acids in the exterior buffer (170). Morever according to Stubbs and Smith (177), the degree of the unsaturation of the fatty acids regulates the affinity and the lost of the membrane receptors. Finally I have the impression that the signal, in its totality, is a well synchronized process in which a movement or an interaction of this or that type of proteins, lipids, carbohydrates or nucleic acids and theirs devivatives are synchronized with the feedback during the propagation of the signal, what one can see even after an incomplete and uncircumstantial information today (180). The phospholipid membrane composition is maintained automatically with a precision that is sufficient for the different enzymes. For instance, according to Mooibrock et al (109), the ration SM/PC is maintained by the action of the sphingomyelinidase. One must remark that the ratio of two branches of the fatty acid utilisation, the synthesis de novo and the exchange are different for the different cell types and for the different experimental conditions (155).

I think that the synchronized process of the increase of the quantity of the unsaturated fatty acids in the phospholipids must serve to other targets. There is the direct modulation of the fatty acids in the interior layer due to the presence of the long fatty acids in the sphingolipid molecules interacting with the receptor in the exterior membrane layer. Recently it was found even the very long chains with 28-38 C atoms in the SM and PC molecules (181-183). The experiment indicating the importance of the transformation of an artificial signal in the case of the long chains done in 1978 (184). I think that for the formation of the bilayer of a more or less equal thickness, the molecules with the shortest fatty chains of one layer must be situated above the longest fatty acids of the another layer. In addition the protein molecules which change the place in the cell during the signal (cytoplasm ⇌ plasma membrane) obtaining "by accident" the shortest fatty acids, could find by such means theirs places in the membrane due to a movement and a fixation (185) in the membrane, which (places) are determined by the receptor carbohydrates, that is the receptor type. For instance, the main substrat of the protein kinase C "obtains" the chain 14:0 in order to be phosphorilated in the membrane (186,187). The protein kinase C (itself) "obtains" the myristic or palmitic chains (188,189).

According to Buss et al (190) the other protein kinases (tyrosine and A) and the phosphatase regulator and apparently all the G proteins are modified with the myristic acid in order to be inserted in the interior membrane layer. Very probably an increase of the quantity of the unsaturated fatty acids serves also to the best reception of these temporary peripheric proteins in the membrane, because the double cys bonds in the fatty acids provoke a more chaotic and less dense conformation in the lipid layer (191). This concerns rather the interior membrane layer because according to Perret et al (192) 90% of AA are situated in the interior layer where the mentioned peripheric temporary components can fit more correctly in agreement with chronology of the signal. The fact that the length of the arachidonic acid even in the extended state will be less than that of the extended oleic acid, according to Li et al (178), argues for this. The fact that, for instance, the phospholipase C and the oncogenes with the activity of the tyrosine kinase have the common domains which are destined apparently for the interaction with the cellular components, indicates the existence of such places with "the adress" (193). There is also the analogous common domains between the phospholipase $A_2$ and the protein kinase C (194). Apparently an increase of the affinity, for instance, of the adenylate cyclase (β-adrenergic receptor), during the inclusion in the membrane of the cys-vaccenic acid, has the relation just with the creation of the particular "adress" for the G protein but not with a diminution of the membrane viscosity (195).

The lysophosphatidyl transferase has an important role in the remplacement of the saturated acids with the unsaturated ones. The activity of such enzyme can be modulated just be the factors which are modified with the chronology of the signal: an increase of the unsaturation degree in the phospholipids in the certain limits /a stimulation of the acyltransferase accompanies an increase of the liberation of the arachidonic acid in the thrombocytes stimulated with the thrombin (196) and in the lymphocytes stimulated with Con A (174)/, and also the change of the concentration of DG. The DG inhibits directly the lysophosphatidyl transferase according to works of Resch and coworkers (197). It is possible to see the support of this in the experiments of Axelrod and coworkers (198) according to which the DG stimulates an increase of the prostaglandin synthesis after the action of the bradykinin with the means which are independent on the protein kinase C. Because if the DG inhibits really the lysophosphatidyl transferase, this must produce an inrease of the arachidonic acid concentration leading to the synthesis of the prostaglandin. The quantity of DG in the membrane diminishes (see above) with the propagation of the signal and a weak concentration of DG in the membrane of the cells belonging to the established cancer lines being in the perma nent process of the signal action (mitosis) testifies for this, where the mentioned DG concentration can be determined easier experimentally (161) (see above).

The experiments with the cyclosporin molecules that inhibited directly (utilizing the control of the activity with the membrane fragments) the activity of the activated acyltransferase during the signal (199) point out in favor of this. Morever the cyclosporin molecule inhibits the inclusion of AA in PC and PE in the lymphocytes, stimulated with Con A and with the antibodies against the immunoglobulin G, and with the $Ca^{2+}$ ionophore A-23187 (152, 199). With this however the inhibition of the inclusion of AA into PI was an exception: in that case there was only the weak inhibition of the inclusion of this acid, confirming elegantly the structure of the additional "semi"-de novo synthesis of PI according to the metabolic branch: DG→→MAG→→PA→→PI (see above). Apparently, the importance of the process of the inclusion of the unsaturated fatty acids in the membrane phospholipid's is confirmed after an inhibition with the cyclosporin molecule of the proliferation of the B and T lymphocytes (151) and the proliferation of the mesanglial kidney cells (200).

A diminution of the affinity of the membrane receptors and their lost from the membrane surface were registed with an increase of the degree of the unsaturation of the fatty acids and in particular with an increase of the quantity of AA (177). As I suppose for the first time, this means that there is an increase of the volume of the zones with a greater fluidity appeared from the begining of the phase separation (49). As a result of the process, these small zones, apparently, flow into a common current in which there is the continuous formation of the patches and the caps. With that, these patches and these caps are constituted within the complexes of the receptors formed due to the association of the exterior "rings" of the patches of SM that makes a dense structure (see above). And later, very probably, the complexes go to the coated pits and disappear from the membrane surface.

For instance in the case of the $\beta$-adrenergic receptors, according to the mentioned publication of Harden et al (81), it was an increase of the maximum of the density gradient belonging to the receptors with a weak affinity after the interaction with the agonist (15 min,37° C). In the case of the $\beta$-adrenergic receptors during the interactuon of the molecules (ligands) with the receptors, there was from begining (with $t_{1/2} \approx 3$ min) the diminution of the affinity of the adenylate cyclase and after (with the lag period 60 min) there was the receptor lost (81,201). In the case, for instance, the receptors with the activation of the tyrosine kinase (EGF,insulin) there was the lost of the receptors during ten or so minutes (202), like in the case of the interaction of the mitogenic lectins or in the case of the antibodies against the immunoglobulins with the lymphocyte surface (203). With this, the processes of the diminution of the mentioned activity in the general plan must take place also from the begining. I repeat, as I propose for the first time, these ten or so minutes of a waiting during the process of the patch formation are necessary just for the increase of the degree of the unsaturation of the fatty acids of these phospholipids in the exterior membrane layer. One can see clearly in the photographs of the publication of Haigler et al (204) that just the complexes of the weak affinity of the EGF receptors with the EGF molecules, which where labeled with the fluorescein and in consequence lost the part of the biological activity, are endocyted better than the native EGF molecules /according to the publication of Schlessinger and coworkers (203), even in the case of the very special precautions to preserve the biological activity of EGF, the derivative activity diminished ( $\approx$ 60%)/. Solely the occupation of the receptors by the ligand, even with an temporary uncoupling of the effector at the level of the regulatory protein or the protein kinase A, is sufficient for the desensibilisation (205,206), that indicates an importance of the primary processes concerning the interaction: ligand-receptor.

Morever the comparison of the different cap types by theirs dimensions, that do not have a direct correlation with the quantity of the receptors,favors the presence and the importance of the lipid part of the complexes. For instance, the quantity of the Con A receptors is 9-27 times greater than the quantity of the receptors of the surface immunoglobulins (207), but the cap formed with the surface immunoglobulins is much greater than the cap formed with the Con A receptors (208,209). And in the case of the numerous molecules of the major surface glycoprotein of the cell which are not the receptors and which are agglomerated by three layers of the antibodies, the cap has the dimension of a little circle (209). One can see from these (and others) experiments that apparently the dimension of the different patches depends strongly on some other factors. Very probably these factors are the lipids constituting the specific complexes with each individual receptor type according to my proposed structure.

Already the first FPR experiments (85) could design the creation of small zones from the begining of the process of the patch formation with the distribution of the visible uniform fluorescence in the fluorescent microscope. The cap (pH neutral) was observed after the labeling of the components of the cell surface (the lipids and the proteins) (weakly alkaline pH). Morever the percentage of the fluorescence recovery was clearly zero, in spite of the fact, that one can see the formation and the mobility of the patches by eye. This paradox of the several years, after this proposed structure, can be ex plained easily with the presence in the begining of the process of the zones of the phase separation much more small than the size of the diameter of the laser beam. The following amalgamation of these zones,apparently during the process of the increase of the lipid unsaturation,led to the process,already visible, of the formation of more and more great patches. The following facts are in favor of this interpretation. When one puts the cells after the process of the capping in the thermostat for the sufficient time,the fluorescent molecules which was not in the cap become mobile (laterally), with the sufficiently homogeneous curve and the diffusion constant similar to that of the proteins in the membrane (85) and having the value of the fluorescence recovery already about 60%.In other words, it is sufficiently clear that the zones formed by the molecules, which become the part of the cap, prevented the fluorescence recovery of these receptors. It seems that the process of the capping was initiated by the glycolipids due to the dimerisation (aggregation) of the dyes at the weakly alkaline pH.Apparently,just this dimerisation,approaching the carbohydrates,was leading to the formation of the hydrogen bonds (see above). These experiments were confirmed with the different receptors in a number of publications (see,for instance,(210)-/.It is possible to find an analogous explanation of the influence of the zones formed by one numerous receptor type,with the ligands, on the movement of the other receptors for the experiments of Poo (211) with the slowing up of the mobility of the free lectin receptors in the electric field after an interaction of the other lectin receptors with their ligands.Very probably the numerous Con A receptors (with the ligand)were having a similar influence on the percentage of the fluorescence recovery for the receptors representing the surface immunoglobulins (212).

In addition to the endocytosis provoked by the occupation of the receptor by the ligand, there is the endocytosis provoked by the phosphorilation of the receptor by the protein kinase C. For instance, in the case of the EGF receptors, where the molecular mechanism of the endocytosis provoked by the ligand and by the protein kinase C is studied better, the initial process of the internalisation is different. There is the phosphorilation of the threonine 654 in the case where the internalisation is provoked only by the protein kinase C, what was not observed in the case of the endocytosis provoked only by the presence of the ligand (71). One can suppose that there is an analogous regulation of the other receptors (less studied in this aspect) by the protein kinase C. This confirms once more the role-key of the protein kinase C in the signal transition in the cell (65 and above).The process,when the protein kinase C can regulate the receptor (that activates it) in eliminating it from the cell surface, is called the homogeneous regulation (65) and I think that this takes place due to the close position of the receptor and the protein kinase C activated by this receptor, that (protein kinase C) is sufficiently immobile (lateral mobility), very probably, in the niche "under" the receptor prepared for this enzyme by the long chains of the exterior layer of the membrane and by the modulation of the interior layer (see above). In addition to this, the protein kinase C can execute the regulation of the other receptors (heterogeneous regulation) in the niches where the protein kinase do not localise (65,66 ). In such regulation, the protein kinase C makes the phosphorilation of more mobile components. Apparently their mobile substrats find (themselfs) the protein kinase C for their phosphorilation with the myristic acid (the cationic subunit of the protein kinase A, the C protein, $p60^{v-src}$).

For instance, in the case of the $\beta$-adrenergic receptors, very probably, the protein kinase C desensibilises the erythrocyte receptors (115) being with them in the same immovable complex. In the case of the $\beta$-adrenergic receptors of the other cells apparently there is no desensibilisation by the protein kinase C. However the protein kinase C in these cells can execute the heterogeneous activation (or the inhibition) due to the phosphorilation of the G protein or the cationic subunit of the protein kinase A (213). One can see this type of the importnat apparently for each signal heterogeneous regulation in the proposed above structure (for instance the activation of the different phospholipases of the interior membrane layer).

Very probably, as I mentioned, the receptors, which must be endocyted, pass by the coated pits, by the endosomes (or GERL) and by the lysosomes (168,214). Generally the receptors of the hormons and the growth factors (the number of theirs activities apriori takes place in the normal bilayer of the plasma membrane) go in the coated pits being already recognized after the interaction with the ligand (215,216). Even that, in the case of the low density lipoprotein (LDL) receptors (which, in general, must furnish only the cholesterol in the cell), changes the value of the lateral diffusion coefficient in $\approx 100$ times after the interaction with the ligand (217) and, for instance, the last results made with the special precausions, indicate the primary diffusive character of their distribution in the membrane (218). In addition after the work of

Goppert-Stübe and Resch (170) one can see that the quantity of the phospholipids rests the same during the action of the signal, with the exception of SM, just such phospholipid that participates in the formation of the lipid complex with the receptor according to the proposed above structure. As it was mentioned,with the addition of DG from outside, the total hydrolysis of SM in the whole cell increases (169), what one can explain also (see above) with the increase of the SM transport into the lysosomes by the coated pits. It is possible to see after the experiments of Wil son et al (219) (see below) the analogous proof of the endocytosis of the lipid complexes with the receptor.

Morever I propose for the first time in my structure that the endocytosis serves only for the feedback of the propagation of the signal to inhibit at least the protein kinase C. It was found recently that the sphingosines inhibit the protein kinase C (220). Because the protein kinase C is the main enzyme, presenting in all signal types (see above), one can wait that the process of the endocytosis /that transports the complexes of the receptors with the sphingolipids (see the proposed above structure) into the lysosomes, where they (sphingolipids) are hydrolysed until the sphingosine/ serves to inhibit the protein kinase C by these sphingolipids transported by this way by the lysosomes in the direction of the interior surface of the plasma membrane.There are also the other results confirming the correctness of this new proposition. According to Maxfield (221), the DNA synthesis increases in 2-7 times after the inhibition of the aggregation of the EGF receptors. According to Kahn (222) an injection of the intact insulin inside the cell do not provoke any biological effect.According to Schlessinger (71), the phosphorilation of the EGF receptors (threonine 654) by the protein kinase C leads to the endocytosis of the receptors and to the inhibition of the mitosis. In addition to this, according to Russel et al (223), the internalisation of the insulin receptors with the antibodies was not capable to make the inclusion of the thymidine in DNA. The following "coincidence" confirms my proposition in an elementary manner.The opioid receptors diminish the cAMP level due to the inhibition of the adenylate cyclase.In this case,apparently,one cannot wait a dangerous decrease (from the biological sense) of the cAMP level and the feedback with the protein kinase C could appear as useless.And in reality the patches formed during the mitosis are not endocyted just in this case and the quantity and the affinity of the active sites do not change apparently because of the formation of disulfide bridges (224), for instance in a different manner with the aggregation and the endocytosis of the TSH receptors stimulating the adenylate cyclase (225). It is shown in the case of the muscarinic receptors (226) that the activity of the protein kinase C rests strong until the receptor inhibition what confirms my conclusion once more. A convinsing proof for my conlusion is the increase of the total quantity of the sphingosine in the cell after an action of the formyl peptides in the neutrophil without its synthesis de novo. The concentration of the sphingosine increases in 1.6 times after the action of the peptides (the 10 nM concentration) and in 2.5 times after the action of the 1 $\mu$M peptide concentration. A similar increase of the sphingosine in the cells was observed after the change for the buffer with $Ca^{2+}$, what must increase the basal level of the endocytosis in this case. Like with the endocytosis there was a remarkable increase only at 37° C but it was absent at 4° C and 25° C (219). The author's interpretation was based on the known presence of the intracellular localisations of the sphingosines (227). Morever the results, obtained with amines, like the chloroquin and monostin moleclules, increasing the pH in the lysosomes and in consequence inhibiting the hydrolysis of the products of the endocytosis and serving for the thesis of the endocytosis importance for the signal action in the cell, are ambigous in reality. Knowing the polar structure of these subtances, one can wait an interference of these substances at the level of the H bonds already during the formation of the complexes of the receptors with the lipids and later at the level of the endocytosis of the receptors. Just the last fact was registed in the scientific literature ˙see for instance (228)/.

That is a more numerous and less affined part of the receptors, as I propose, serves for the negative regulation at least of the protein kinase C. At the same time a less numerous and more affined part of the receptors must rest on the cell membrane surface. The necessity of the permanent presence of these receptors on the membrane surface was demonstrated convinsingly in the experiments of Cautricasas and coworkers (229) according to which the presence of these EGF receptors for the execution of the mitosis was necessary during at least 8 hours.

Very probably these receptors are indispensible for the sustained current of $Ca^{2+}$ through the plasma membrane (see above) (79,230). The fact, that the concentration of the ACTH hormon which is necessary for the half of the maximal $Ca^{2+}$ current would be in 14 times greater than that for the half of the maximal aldosteron production, point out in favor of this (231). As the confirmation of the structure of the universal action of the $Ca^{2+}$ current for all the signal types proposed above, its activity was registed after the action of the hormons with the primary activation of the adenylate cyclase and the activation of the PPI cycle (232).

Apparently the PA has the hole-key in the sustained $Ca^{2+}$ current. One can remark the perallelism between the PA formation and the $Ca^{2+}$ current through the membrane and their mutual regulation. According to Takuwa et al (233) an increase of a basal level of the susutained $Ca^{2+}$ current begins after $\approx 5$ seconds as well as the stimulation of the PA synthesis (103,112,234) that makes the correlation with the signal action (235). The publication of Putney et al (236) indicates the role of PA and $Ca^{2+}$ in the action of the signal. According to this publication, the PA stimulated the signal only in the presence of $Ca^{2+}$. Morever the affinity between $Ca^{2+}$ and PA (and also the $Ca^{2+}$ ionophore A 23187) was well shown (comparing with other phospholipids).

The fact that the regulation of the quantity of PA during the transformation of PA into PI in the membrane would be realized by the two second messengers cAMP amd $Ca^{2+}$ (146) having the opposite effects (the $Ca^{2+}$ increases the PA concentration), indicates the role-key of PA.

I would like to propose here the molecular structure, which is new in principle and very important, of the sustained $Ca^{2+}$ current through the membrane: I think that there is an increase of the quantity of the lysoPA in the interior membrane layer after the actions of the phospholipases $A_2$ activated with the structure of the signal /morever with the presence of the specific, just for PA, phospholipases $A_2$ (235)/ in the exterior membrane layer and there is a moderated activation of the acyltransferases due to an increase of the quantity of DG with the signal (see above). With this, the numerous structures of the closed micellar type (237) must be formed, that favors the passage of lysoPA in the membrane interior layer where once more there is the reacylation of lysoPA with an exit of this structure in the different directions in the plane of the interior layer. It passes something like the rotation of the "wheel", constituting this membrane lipid phase with the inclusion of the phospholipids from the exterior layer and their exit from the "wheel" in the interior layer. One can suppose that the specific for PA phospholipase $A_2$ is situated near the base of this structure in the zone of these receptor complexes with the strong affinity. This proposition can take place for the acyltransferase (199). The bivalent $Ca^{2+}$ with the strong affinity for PA adhering to lysoPA on the exterior surface, by such means crosses the membrane in rotating with lysoPA in the "wheel" towards the interior membrane surface. With this, the $Ca^{2+}$ must form also the lipid bridges strengthening the neighbouring phospholipids in the "wheel". I think that just this phase is the base of the action of a common structure of the creation of the relatively narrow pores during the action of the different lytic agents (238), and the bivalent cations, the $C_a^2{}^+$ included, diminish the membrane lysis very probably due to the mentioned strengthening of the phospholipids in this phase. According to Lapetina and Cautricasas (239) there was $\approx 10\%$ of lysoPA during the signal action in the thrombocytes. The experiments concerning the transformation of PA into PI after the lysoPA action (146) confirm brilliantly the $Ca^{2+}$ transport by these phospholipids because the $Ca^{2+}$ inhibits this transformation (see above).In this general case as a result of my proposed structure, the $Ca^{2+}$ rests with its carrier (undergone to the acylation) on the interior surface of the membrane without the change of the total $Ca^{2+}$ concentration in the cell interior but with the change of the local surface concentration in the interior membrane layer, what intails an excellent agreement with the conclusions of Alkon and Rasmussen (240).

Basing on this result, I have the possibility to deduce that the cellular mobility is determined by an appearance of these new micellar structures of the another phase after the activation of the receptors and their rotation like the chenilles of the tractor. The fact, that the rate of the neutrophil mobility after the action of the formyl peptides (and only after their action due to the creation of the structured complexes, see above) increases after the exterior application of the phospholipases (241) is convincing in favor of this. Apparently there is an other confirmation due to an inhibition of the acyltransferase in the interior layer after the action of the phorbol ester, that stimulates the increase of DG in the interior layer with the protein kinase C (see above) in inhibiting after this the acyltransferase of the interior layer. The reversibility of the cellular mobility after the action of a DG kinase inhibitor (241) permits to see a sufficiently picturesque image of the rotation of the "wheels" of the mobility in the opposite sense because with the increase of the DG concentration of the interior layer, the lysoPA molecules can be accumulated already in this layer due to the inhibition of the acyltransferase and move with the "wheel" in the direction of the exterior layer.

A presumed important role of PA in the activation of the phospholipases $A_2$ of the interior and exterior layer was underlined earlier. The serie of the experiments concerning the action of $Ca^{2+}$ and cAMP on the reaction of a transformation of PA into PI confirms this plan (51,146,239,242). According to Nishizuka (114) the PC (like SM) inhibits the protein kinase C. This fact is supported by an inclusion of 80-90% of the myristic acid /serving (see above) like the "adress" for the substrates of the protein kinase C/ in PC (102,136). It can be suggested that PA /the myristic acid incorporates also in PA-(103)/, the phosphatidylserine molecule and DG concur with PC for a strong activity of this enzyme-key.

One can apply this conclusion of the role-regulator on the endocytosis to the important problem of the immunology- the interaction between the B and T lymphocytes resulting in the formation of the cells which synthetize the immunoglobulin C (IgC). According to Zagyansky (243-245) during the interaction between the lymphocytes B (with the receptor for the antigen- the membrane IgM) and the T lymphocytes (with the receptors for the antigen), the interaction between the fragments of the antigen, which become a bridge between the B and T lymphocytes and the membrane IgG takes place. As the result the IgC forms the "scorpion" structure. This membrane IgG molecule must react later with the Fc receptors for the antibodies of the B lymphocytes which arise as a result of the action of the lymphokine molecules secreted by the T lympho cytes in the microsurrounding of the B cells. The possibility of this directed secretion by the T lymphocytes during the endocytosis was confirmed recently (246) . I think that after this interaction there is the endocytosis (247) of the antigen by the B lymphocytes with the proteolysis of an antigen in the lysosomes.After this proteolysis,the fragments of the antigen must move in the direction of the membrane (likewise the mentioned above products of the hydrolysis of the sphingolipids),without or with a preliminary association in the cytoplasm with the products of the major hystocompatibility complex II (MHC II) that must appear as a result of the action of the B cell growth factor (BCCF) that induces the appearance of this MHC class in B cells (248,249) .I think that after this phase there is the activation of the B lymphocytes (250) and the first proliferation of the cells takes place.Very probably there is only one division at this stage (250)-.After this division, as I propose,the second interaction between the B and T lymphocytes takes place.This time the B lymphocytes are already the cells which present the antigen (251-254) .This second interaction between the B and T cells takes place already when there is the special change of the T cell receptor complex /the CD4 molecule interacts with the receptors of the T cells (255)-/.And the commutation of the B cells for the synthesis of the IgC subclasses with the consequent proliferaion and differenciation passes already after the interaction of the B and T lymphocytes with the MHC II complex (250).

That is the described above innovation of the new structure of the action of the hormons, the neurotransmetters and the growth factors gives the sequence of the general correlation of events which take place during the signal. Without doubt, there will be the sequence of the interactions of such or such particular carbohydrates or lipids. With the exact establishment of such sequences (Maps) for the particular receptor by the methods of the molecular and cellular biology as the chemical bivalent crosslinking, the energy migration, the group blocking of the antibodies, the exact chemical modification of the deuterium derivatives in such or such conditions depending on a tissue, an age or a sex, the conditions never existed will be presented for the Medicine of tomorrow, because, without any doubt, there are the derangements of this process in the normal state leading to many diseases. Certainly with this, the target of the utilisation of the new preparations is the approachment of the state of the action of the receptors in the pathological case to their action in the normal case due to the introduction of the preparations of the gangliosides and the neutral glycolipids in an organism. Fortunately, for instance, the gangliosides incorporate easily in the membrane of the native cells from the exterior solution (2,256,257). The empiric fact that with an external addition of the gangliosides, there is an increase of the action of the different protein kinases (258,259) indicates the exactness of the chosen way, according to the fundamental structure.

After a number of works, one knows that the different modulations of the cell growth lead to the change of the glycolipid composition (5,260,261). The change of the glycolipid composition is brilliantly observable from the numerous comparisons of the normal and cancer cells (262-265). The essential difference in the process of the fluorescence recovery was shown between the normal and cancer cells (266,267) what must testify apparently in favor of the different dimensions of the zones of the restricted mobility of the receptors with the ligands in the case of normal and cancer cells reflecting the different composition of the lipid components in the complex. Morever the empiric works concerning an addition of the glycolipids to the cell membrane showed the considerable changes in the most essential processes in the cells, and in particular an arrest of the cell proliferation (2,256,263,268). In consequence one can see well from these works the role of the gangliosides in the process of the uninterrupted division of the cells and the possibility with their aid to influence this process. Of course, it is only with the scientifique knowledge of the sequence of the events during the formation of the complex: receptor → sphingolipids → phospholipids in the agreement with the proposed structure,one can find the most effective preparations of the natural sphingolipids or the synthesized specially sphingolipids for this target by the classical methods of the biochemistry or the organic chemistry (269).

An other important application for the medicine, followed from the fundamental structure, concerns ten or so mental diseases of the new-borns (sphingolipidoses) (notably the diseases of Sandhoff, of Fabry, of Caucher, of Krabb, of Niemann-Pick, of Tay-Sachs, GM3 gangliosidosis, metachromatic leucodystrophy,generalised gangliosidosis,lactosylceramidosis).These sphingolipidoses are the hereditary diseases with the accumulation of the lysosphingolipids due to the perturbed catabolism that leads to the sphingosine. According to Hunnun and Bell (220) and Kreutter et al (270), the different gangliosides and their derivatives inhibited the protein kinase C in vitro.This first group of the authors attributes to "intracellular accumulation of the lysosphingolipids...inhibition of protein kinase C". The fact of the presence of such accumulation in the cell, as the authors state this, serves to their hypothesis (227). The second group of the authors attributes this inhibition to the reaction of the protein kinase C with the gangliosides. My fundamental structure gives an other, clear, explanation of the form of the regulation of the protein kinase C. The sphingolipids are found in the lysosomes as a result of the endocytosis provoked by the ligand, what follows for the first time from my proposed structure. In the normal case there is their hydrolysis until the sphingosine that moves by the intracellular "adressed" ways in the direction of the interior membrane surface like the feedback for the protein kinase C (see above). I propose for the first time that the inactivation of the kinases during their interaction in vitro with the other sphingolipids and their products passes only because of the similarity of their structure with one of the sphingosine but these glycosphingolipids cannot be transported by the same way as the sphingosine. In consequence, I propose to introduce the sphingosine to struggle against the mentioned diseases of the derangements of the sphingosine catabolism by the bias of the endocytosis - (and only the endocytosis by the coated pits) because the empiric introduction of the sphingolipids, applied by authors (220) must lead to a number of other effects (according to my structure), and their delivery to the interior membrane layer cannot be guaranteed adequately. I state that one can, for instance, create the new preparation with the method of the extraction with the heptan of the lipids of LDL (271), endocyted easily, and their remplacement with the sphingosine.

The creation of the more effective and less nocuos preparations for the execution of the anaesthesia is the important application of the described structure because according to this structure the mechanism of the anaesthetic action (see above) consists to destroy the H interlipid bonds during the formation of the complex: receptor → sphingolipids → phospholipids → cholesterol due to the polar groups of the anaesthetic.

It is known that the Human immunodificiency virus (HIV), covered with the antibodies, still could be endocyted by the cells through the coated pits (272) and the infection is not arrested (273).The interactions of the other viruses follow often the same destiny (274-276).Taking into consideration that the H bonds and the charges have the role-key in the endocytosis (see above) ,I propose to utilise against the viruses the antibodies with the modified charge which must prevent the endocytosis of these viruses (evidently in the case where the binding of the immodified antibodies is not prevented).Certainly this extra charge can also help to prevent the virus attachment to the cell. For the mentioned charge modification,one can use the antibodies with the modified sequence with the increased quantity of the charged amino acids-(especially in the Fc fragment), or one can attach to the antibody (especially in the Fc fragment) the different charged molecules (for instance, polyglutamic acid or polylysine).These extra charges,according to the proposed structure,must disturb the endocytosis of the viruses.One can wait also,during an utilisation of the same types of the charged molecules,the change of the vital activities of the bacteries conducting to their neutralisation.For instance the arrest of the mitosis:because apparently a phase separation depending on charges must take place during the mitosis (see above).

Literature

1.Karplus M. & McCammon J.A. Annu.Rev.Biochem.52,263,1983

2.Bremer E.G. et al J.Biol.Chem.259,6818,1984

3.Sharom F. & Grant C.W.M. Biochim.Biophys.Acta 507,280,1978

4.Järnefelt J. et al Trends Biochem.Sci.3,110,1978

5.Hakomori S. Annu.Rev.Biochem.50,733,1981

6.Jeffrey G.A. & Matra J. Acta Cryst.B 39,469,1983

7.Goodby J.M. Molec.Cryst.Liquid Cryst.110,205,1984

8.Marsh D. et al Biochemistry 27,2398, 1988

9.McDaniel R. & McLaughein S. Biochim. Biophys.Acta 819,153, 1985

10.Bach D. et al Chem.Phys.Lipids 31,381,1982

11.Spiegel S. et al J.Biol.Chem.99,1575,1984

12.Feizi T. et al Biochem.J.173,245,1978

13.Curatolo W. Biochim.Biophys.Acta 906, 137,1987

14.Goins B. et al Biophys. J.49,849,1986

15.Kanfer J.M. et al J.Biol.Chem.251,7610,1976

16.Edelman G.M. et al Proc.Natl.Acad.Sci.USA 70,1442,1973

17.Thompson T.E.et al Biochim. Biophys.Acta 817,229,1985

18.Osawa T. & Berri M. Methods Enzymol.150,147,1987

19.Zagyansky Y. & Jard S. Exp.Cell Res.132,387,1981

20.Armstrong G.D. J.Biol.Chem.263,8677,1988

21.Pascher I. Biochim.Biophys.Acta 455,433,1976

22.Barenholz Y. et al Biochemistry 15,2441,1976

23.Estep T.N. Biochemistry 19,20,1980

24.Freire E. Biochemistry 19,3662,1980

25.Boggs J.M. Biochim.Biophys.Acta 906,353,1987

26.Sonnino S. et al Chem.Phys.Lipids 42,3,1986

27.Curatolo W. Biochim.Biphys.Acta 906,111,1987

28.Huang S.B. et al Biochemistry 22,4756,1983

29.Kerlavage A.R. et al Trends Pharm.Sci.8,426,1987

30.Dolman H.C. et al Biochemistry 26,2657,1987

31.Goldstein J.L. et al Annu.Rev.Cell.Biol.1,1,1985

32.Block L.A. & Pletcher A. Trends Pharm.Sci.9,214,1988

33.Curtain C.C. et al Biochim. Biophys.Acta 596,43, 1980

34.Sedlacek H.H. et al FEBS Lett.61,272,1976

35.Tkaczuk P. & Thornton E.K. Biochem. Biophys.Res.Comm. 91 1415,1979

36.Crook S.J. et al Biochemistry 25,7488,1986

37.Curtain C.C. Biomembranes 12,229,1984

38.Curtain C.C. Methods Ensymol.150,480,1987

39.Barenholz Y. & Thompson T.E. Biochim.Biophys.Acta 514,239,1978

40.Bruzic K.S. Biochim.Biophys.Acta 939,315,1988

41.Khare R.S. & Warthington C.R.W. Biochim. Biophys.Acta 514,239, 1978

42.Schmidt C.F. et al Biochemistry 16,2649,1977

43. Barenholz Y. & Thompson T.E. Biochim.Biophys.Acta 604,129,1980

44.Schlessinger J. J.Cell.Biol.103,2067,1986

45.Cochet C. et al J.Biol.Chem.263,3290,1988

46.Lund-Katz S. et al Biochemistry 27,3416,1988

47.Stevens V.L. et al Biochemistry 25,4287,1986

48.Chapman D. in"Membrane Reconstitution", Poste G. and Nicilson G.L. eds, 1-41,1982,Elsevier Biomed.Press

49. Zagynansky Y. & Jard S. Nature 280,591,1979

50.Van Dijck P.W.M. et al Biochim.Biophys.Acta 455,576,1976

51.Broekman M.J. et al J.Clin.Invest.66,275,1980

52.Schick P.K. et al Biochim.Biophys;Acta 556,434,1979

53.Schroeder F. Prog.Lipid Res.23,97,1984

54.Williams L.T. & Lefkowitz R.J. J. Biol.Chem.252,7207,1977

55.Khorana H.G. J.Biol.Chem.263,7439,1988

56.Wong S.K.F. et al J.Biol.Chem.263,7925,1988

57.Keller D.J. et al J.Phys.Chem.90,2311,1986

58.Fong J.W. Lipids 12,857,1977

59.Brockerhoff H. Lipids 9,645,1974

60.Huganir R. & Greengard P. Trends Pharm.Sci.8,472,1987

61.Morrison A.R. & Needelman P. in "Hormonal Function and the Kidney",Brenner B.M. and Stein J.H. eds, vol.4,66-114,1979-citated by (63)

62. Axelrod J. et al Trends Neurosci.11,117,1988

63.Yamaguchi D.T. et al J. Biol.Chem.263,10745,1988

64.Delphin A.C. Trends Neurosci.11287,1988

65.Berridge M.J. Annu.Rev.Biochem.56,159,1987

66.Enna S.J. & Karbon E. Trends Pharm.Sci.8,21,1987

67.Alkon D.L. & Rasmussen H. Science 239,988,1988

68.Michel B. Trends Pharm.Sci. 9N 4,VI,1988

69.Peralta E. Nature 334,434,1988

70.Kojima I. et al J. Biol.Chem.260,9171,1985

71. Schlessinger J. Biochemistry 27,3119,1988

72.Meldolesi J. & Pozzon T. Exp.Cell.Res.171,271,1987

73.Frelin C. et al Eur.J.Biochem.174,3,1988

74.Berridge M.J. Ann. N.Y.Acad.Sci.494,39,1987

75.Lefkowitz R.J. et al Ann.N.Y. Acad.Sci.185,195,1971

76.Jard S. et al Adv.Cycl.Nucl.Res.5,31,1975

77.Kahn C.R. J.Cell.Biol.70,261,1976

78.Bonne D. & Cohen P. Eur.J.Biochem.56,295,1975

79.Yanagibushi K. et al Endocr.Jpn.25,545,1978

80.Muchmore D.B. et al J.Biol.Chem.256,365,1981

81.Harden T.K. et al. Science 210,441,1980

82.Chorev M. et al Eur.J.Biochem.146,9,1985

83.Edidin M. in "New Comprehensive Biochemistry",vol.1 Membrane Structure p.37 eds Finean J.B. and Michel R.H.,Elsevier,1981

84.Jacobson K. Annu.Rev.Physiol.49,163,1987

85.Edidin M., Zagyansky Y. & Lardner T.J. Science 191,466,1976

86.Poo M. et al J.Cell.Biol.76,483,1978

87.McCloskey M.A. et al J.Cell.Biol.99,778,1984

88.Tank D. et al J.Cell.Biol.101,148,1985

89.De Laat S.W. et al Proc.Natl.Acad.Sci.USA 77,1526,1980

90.Stiles G.L. et al J.Biol.Chem.259,8655,1984

91.De Meyts P. et al J.Biol.Chem.251,1877,19767

92.Carpenter G. & Cohen S. Biochem.Biophys.Res.Comm.79,545,1977

93.Catt J. et al Nature 280,109,1979

94.Pratt R.& Pastan I. Nature 272,68,1978

95.Lefkowitz R.J. & Caron M.G. J.Biol.Chem.263,4993,1988

96.Low H.G. & Satiel A.R. Science 239,268,1987

97.Satiel A.R. et al Phil.Trans.R.Soc.Lond.B 320,345,1988

98.Margolis R.K. et al Biochemistry 27,3454,1988

99.Satiel A.R. et al Science 233,967,1986

100.Moto J.M. et al J.Biol.Chem.262,2131,1987

101.Standaert M.L. et al J.Biol.Chem.263,8696,1988

102.Farese R.V. et al. Science 236,586,1987

103.Billah M. et al Biochem.Biophys.Res.Comm90,92,1979

104.Cockoroff S. Biochim.Biophys.Acta 795,37,1984

105.Honeycutt P.J. & Niedel J.E. J.Biol.Chem.261,15900,1986

106.Griendling K.K. et al J.Biol.Chem.261,5901,1986

107.Exton J.H. FASEB J. 2,2670,1988

108.Ono T. et al J.Biol.Chem.263,5732,1988

109.Mooibroek M.J. et al J.Neurochem.44,1551,1985

110.Brockerhoff H. et al Lipids 21,405,1986 111.Hirata F. Proc.Natl.Acad.Sci.USA 78,3190,1981

112.Lapetina E.G. & Cautricasas P. Biochim.Biophys.Acta 573,394,1979

113.Downes C.P. Trends Neurosci.11,336,1988

114.Nishizuka Y. Nature 308,693,1984

115.Levitzki A. Physiol.Rev.66,819,1986

116.Sibley D.R. et al Biochem.Biophys.Res.Comm.121,973,1984

117.Kelleher D.J. et al Proc.Natl.Acad.Sci.USA81,4316,1984

118.Jacob S. Proc.Natl.Acad.Sci.USA 80,6211,1983

119.Iwashita S. & Fox C.F. J.Biol.Chem.259,2559,1984

120.Bell J.D. et al J.Biol.Chem.260,2625,1985

121.Bollag G.E. et al Proc.Natl.Acad.Sci.USA 83,5821,1986

122.Yarden Y. & Ulrich A. Biochemistry 27,3113,1988

123.Nishizuka Y. Nature 334,661,1988

124.Clegg R.A. Curr.Top.Cell.Regul.29,77,1988

125.Siffert W. & Akkerman J.W. Trends Biochem.Sci.13,148,1988

126.Hagag N. et al Mol.Cell.Biol.7,1984,1987

127.Zavoiko G.V. et al J.Biol.Chem.261,13160,1986

128.Rozengurt E. Ciba Found.Symp.116,66,1985 (Pitman,London)

129.Grillone L.R. J.Biol.Chem.263,2658,1988

130.Griendling K.K. et al J.Biol.Chem.263,10620,1988

131.Pai J.K. et al Biochem.Biophys.Res.Comm.150,355,1988

132.Irving H.R. & Exon J.H. J. Biol.Chem.262,3440,1987

133.Wolf R.A. & Gross R.W. J.Biol.Chem.260,7295,1985

134.Bocikino S.B. et al J.Biol.Chem.260,14201,1985

135.Bocikino S.B. et al J.Biol.Chem.262,15309,1987

136.Cabot M.C. et al FEBS Lett.233,153,1988

137.Besterman J.M. et al Proc.Natl.Acad.Sci.USA 83,6785,1986

138.Reibman J. et al J.Biol.Chem.263,6322,1988

139.Augert G. & Exton J.N. J.Biol.CHem.263,3600,1988

140.Yap W.H. et al Science 234,355,1986

141.Schonhardt T. & Ferber E. Biochem.Biphys.Res.Comm.149,769,1987

142.Loeb L.A. & Gross R.W. J.Biol.Chem.261,10467,1986

143.Hirata F. J.Biol.Chem.256,7730,1981

144.Touqui L. et al Nature 321,177,1986

145.Flesch I. & Ferber E. Biochim.Biophys.Acta 889,6,1986

146.Lapetina E.G. et al J.Biol.Chem.256,11984,1981

147.Bell R et al Proc.Natl.Acad.Sci.USA 76,3238,1979

148.Habernicht A.J. et al J.Biol.Chem.256,12329,1981

149.Putney J.W. et al Phil.TRans.R.Soc.Lond.B 296,37,1981

150.Billah M.M. et al J.Biol.Chem.256,5999,1981'

151.Kroggel R. et al Immunobiol.75,159,1987

152.Szamel M. et al Cell.Immunol.93,239,1985

153.Irvin R.F. Biochem.J.204,3,1982

154.Spector A.A. et al Prog.Lipid Res.19,155,1981

155.Rosenthal M. Prog.Lipid Res.26,87,1987

156.Scow R. & Blanchette E. Prog.Lipid Res.24,197,1985

157.Howard B.V. & Kritchevsky D. Lipids 5,49,1970

158.Hülsmann W.C. et al Biochem.Biophys.Res.Comm.102,440,1981

159.Debeer L.J. et al J.Biol.Chem.254,8841,1979

160.Groener J.E.M. Knauer T.E. Biochim.Biochys.Acta 665,306,1981

161.Mountford C.E. & Wright L.C. Trends Biochem.Sci.13,172,1988 .

162.Vance D.E. & Choy P.C. Trends Biochem.Sci.4,145,1979

163.Rosoff P.M. et al Cell 54,73,1988

164.Bourn H.R. Cell 53,669,1988

165.Keen J.H. & Black M.M. J. Cell Biol.102,1325.1986

166.Larkin J.M. et al Cell 33,273,1983

167.Kojima I. et al J.Biol.Chem.260,9171,1985

168.Pearse B.M.F. & Crowther R.A. Annu.Rev.Biophys.Biophys.Chem.16,49,1987

169.Kolesnick R. J.Biol.Chem.262,16759,1987

170.Goppert-Strübe M. & Resch K. Biochim.Biophys.Acta904,22,1987

171.Rode H.M. et al Biochim.Biophys.Acta 688,66,1982

172.Szamel M. & Resch K. J.Biol.Chem.256,11618,1981

173.Averdunk R. & Lauf P. Exp.Cell Res.93,331,1975

174.Ferber E. Biomembranes 12,633,1984 (R)

175.Poon R. et al Biochim.Biophys.Acta640,58,1981

176.Szamel M. & Resch K. J.Biol.Chem.256,9198,1981

177.Stabbs C.D. & Smith A.D. Biochim.Biophys.Acta 779,89,1984

178.Li A.G. et al Prog.Lipid Res.25,41,1986

179.Hochman Y. et al J.Biol.Chem.258,6509,1983

180.Parker C. Methods Ensymol.150,29,1987

181.Poulos A. et al Biochem.J.240,891,1986

182.Poulos A. et al Biochem.J.248,961,1987

183.Poulos A. et al Biochem.J.253,645,1988

184.Schmidt C.F. et al Nature 271,775,1978

185.Rein A. et al Proc.Natl.Acad.Sci.USA 83,7246,1986

186.Buss J.E. et al J.Virol.58,468,1986

187.Aderem A.A. et al Nature 332,362,1988

188.Sefton B.M. & Buss J.E. J.Cell Biol.104,1449,1987

189.Mochly-Rosen D. & Koshland D.F. J.Biol.Chem.262,2291,1987

190.Buss J.E. et al Proc.Natl.Acad.Sci.USA 84,7493,1987

191.Seeling A. & Seeling J. Biochemistry 16,45,1977

192.Perret B. et al Biochim.Biophys.Acta 556,434,1979

193.Suh B.G. et al Proc.Natl.Acad.Sci.USA 85,5419,1988

194.Maraganore J. Trends Biochem.Sci.12,176,1987

195.Henis Y.I. et al J.Biol.Chem.257,1407,1982

196.Rittenhouse-Simmons S. et al Biochem.Biophys.Res.Comm.70,295 ,1976

197.Goppert-Strübe M. et al Biochem.J.247,773,1987

198.Burch R.M. et al J.Biol.Chem.263,4764,1988

199.Szamel M. et al J.Immunol.136,264,1986

200.Martin M. et al Biochem.Pharmacol.37,1083,1988

201.Stadel J.M. et al J.Biol.Chem.258,3032,1983

202.Schlessinger J. et al Proc.Natl.Acad.Sci.USA 75,2659,1978

203.Unanue E.R. Adv.Immunol.24,37,1976

204.Haigler H. et al Proc.Natl.Acad.Sci.USA 75,3317,1978

205.Green D.A. & Clark R.B. J.Biol.Chem.256,2105,1981

206.Shear M. et al J.Biol.Chem.251,7572,1976

207.Stackpole C.W. J.Cell.Physiol.83,441,1974

208.Kourilsky F.M. et al Eur.J.Biochem.2,249,1972

209.Bourgignon L.Y.W. & Bourgignon G.J. Int.Rev.Cytol.87,195,1984

210.Drugstein P. et al Proc.Natl.Acad.Sci.USA 76,6163,1979

211.Poo M. Annu.Rev.Biophys.Bioeng.10,245,1981

212.Henis Y.I. & Elson E.L. Proc.Natl.Acad.Sci.USA 78,1072,1981

213.Bell J.D. et al J.Biol.Chem.260,2625,1985

214.Hopkins C. Trends Biochem.Sci.11,473,1986

215.Maxfield F. et al Cell 14,805,1978

216.Carpentier J.L. et al J.Biol.Chem.95,73,1982

217.Tank D.W. et al J.Cell.Biol.101,148,1985

218.Kukuda S. et al Virc.Arch.B. Cell Pathol.52.1,1986

219.Wilson E.W. et al J.Biol.Chem.263,9304,1988

220.Hunnun Y.A. & Bell R.M. Science 235,670,1987

221.Maxfield F. Proc.Natl.Acad.Sci.USA76,5731,1979

222.Kahn R. Annu.Rev.Med.36,429,1985

223.Russel D.S. et al J.Biol.Chem.262,11833,1987

224.Hazum E. et al Proc.Natl.Acad.Sci.USA 77,3088,1980

225.Avivi A. et al Science 214,1237,1981

226Liles W.C. et al J.Biol.Chem.261,5307,1986

227.Symington F.W. et al J.Biol.Chem.262,11356,1987

228.Janicot H. et al Biochem.J.253,735,1988

229.Shecter Y. et al Proc.Natl.Acad.Sci.USA 75,5788,1978

230.Kojima I. et al J.Biol.Chem.260,9177,1985

231.Kojima I. et al J.Biol.Chem.260,4248,1985

232.Mauger J.P. et al J.BiolChem.260,11635,1985

233.Takuwa N. et al J.Biol.Chem.262,182,1987

234.Salmon D.M. & Honeyman T.W. Nature 284,344,1980

235.Billah M.M. et al J.Biol.Chem.256,5359,1981

236.Putney J.W. et al Nature 284,345,1980

237.Cullis P.R. & de Kruijff B. Biochim.Biophys.Acta 559,399,1979

238.Bashford C.L. et al J.Biol.Chem.261,9300,1986

239.Lapetina E.G. & Cautricasas P. Nature 292,367,1986

240.Alkon D.L. & Rasmussen H. Science 239,998,1988

241.Lackie J.M. J.Cell.Sci.89,449,1988

242.Kowalska M.A. et al Biochem.J.253,255,1988

243.Zagyanksy Y. Bull.Offic.Propr.Indust."Brevets" n° 28,1988,Application N° 2 609 398

244.Zagyansky Y. Gazette of PCT N° 21,1988, Application for the International PCT Patents, Publication N° WO 88/06837

245.Zagyanksy Y. "Premature Scientific Discovery? Today physics and genetics speak the same language concerning the sudden appearance of a flexible hinge region in immunoglobulins during progressive evolution of vertebrates", ISBN: 2-9502914-0-6, dépôt legal N° 13314-30.05.1988,Paris 1988.

246.Poo W.J. et al Nature 332,3781,1988

247.de Franco A.L. Nature 334,199,1988

248.Lee F. et al Proc.Natl.Acad.Sci.USA 83,2061,1986

249.Noma Y. et al Nature 319,640,1986

250Jelinek D.F. & Lipsky P.E. Adv.Immunol.40,1,1987

251.Tony H.P. & Parker D.G. J.Exp.Med.161,223,1985

252.Lanzavecchia A. Nature 314,537,1985

253.Abbas A.K. et al J.Immunol.135,1661,1985

254.Mosier D. J.Immunol.136,2090,1986

255.Kupfler A.et al Proc.Natl.Acad.Sci.USA 84,5888,1987

256.Bremer E.G. et al J.Biol.Chem.261,2434,1986

257.Hanai N. et al J.Biol.Chem.263,10915,1988

258.Ledeen R.W. J.Neurosci.Res.12,147,1984

259.Tsuji S. et al Biochem.(Tokyo) 97,969,1985

260.Flshman P. et al Biochem.Biophys.Res.Comm.59,292,1974

261.Patt L. et al Nature 273,379,1981

262.Holmes E.H. et al J.Biol.Chem.262,15649,1987

263.Dyatlovskaya E.V. & Bergelson L.D. Biochim.Biophys.Acta 907,125,1987

264.Matyas G.R. et al Proc.Natl.Acad.Sci.USA 84,6065,1987

265.Prokazova N.V. et al Eur.J.Biochem.172,1,1988

266.Zagyanksy Y. & Edidin M. Biochim.Biophys.Acta 433,209,1986

267.Zagyanksy Y. et al FEBS Lett.77,206,1977.

268.Laine R.A. & Hakomori S. Biochem.Biophys.Res.Comm.54,1039,1973

269.Methods Enzymol.vol.138,Ed.Ginsburg V.,1987

270.Kreutter D. et al J.Biol.Chem.262,1633,1987

271.Anderson R.G.W. et al J.Recep.Res.1,17,1980

272.Pausa C.D. & Price T.M. J.Cell Biol.107,959,1988

273.Scientific American 259,N° 4,1988

274.White J. et al Quart.Rev.Biophys.16,151,1983

275.Dimmock N.J. J.Gen.Virology 65,1015,1984

276.Dimmock N.J. Trends Biochem.Sci.12,70,1987

## Claims

1.Innovation "The fundamental structure of the action of the hormons, the neurotransmetters and the growth factors in the transmission of a signal in the cell and its very important consequences" is explained, wherein it comprises the serie of the new knowledges which permitted to establish the new structure of the action of the hormons, the neurotransmetters and the growth factors with the following new validations:
the creation of the complexes: ligand → receptor → lipids according to the plan: receptor → ganglioside → cerebroside → sphingomyelin → cholesterol especially with the intercarbohydrate and interlipid hydrogen bonds;
the modulation of the structure of the interior membrane layer for the creation of the sites of the association with the mobile proteins like the protein kinase C, the G proteins or the phospholipases with the very long chains of the exterior layer and an increase of the unsaturation of the fatty acids of the interior layer;
the validation of the endocytosis as the means of the negative regulation (feedback) of the enzymes of the interior membrane layer, in particular the protein kinase C;
the validation of the micellar and rotational structure for the $Ca^{2+}$ transport through the plasma membrane with the lysophosphatidic acid after the action of the phospholipase $A_2$ and the acyltransferase; the conclusions and the practical consequences made from this structure.

2.Innovation as defined in claim 1, wherein the new preparations with the sphingosine against the mental diseases- sphingolipidoses (diseases of Sandhoff,of Fabry, of Gaucher, of Krabb, of Niemann-Pick, of Tay-Sachs, $GM_3$ gangliosidosis, metachromatic leucodystrophy, generalised gangliosidosis, lactosylceramidosis) incorporating the carriers of the sphingosine which could be endocyted in the cell by the coated pits.

3.Innovation as defined in claim 1, wherein the new preparations for the execution of the anaesthesia which eliminate, due to their polar groups, the intermolecular hydrogen bonds after the creation of the complex: receptor → → sphingolipids → phospholipids → cholesterol.

4.Innovation as defined in claim 1, wherein the new preparations of the natural and synthetic sphingolipids against the cancer which block the continuous division of the cell.

5.Innovation as defined in claim 1, wherein the new preparations of the gangliosides and the neutral glycolipids which approach the action of the receptors in the pathological state to these in the normal state known with the help of the sequences (Maps) of the action of each particular receptor in the normal and the pathological state.

6. Innovation as defined in claim 1, wherein the new preparations of the antibodies of any type and any origin with the modified charge are proposed against all viruses (the HIV virus included) which must prevent their penetration into the cell and against the bacteries which must prevent their division.

**DECLARATION**
which under Rule 45 of the European Patent Convention shall be considered, for the purpose of subsequent proceedings, as the European search report

European Patent Office

Application number

EP 89 10 4884

| The Search Division considers that the present European patent application does not comply with the provisions of the European Patent Convention to such an extent that it is not possible to carry out a meaningful search into the state of the art on the basis of all claims.<br>Reason: | CLASSIFICATION OF THE APPLICATION (Int. Cl.4) |
|---|---|
| Scientific theory: EPC Article 52(2)(a); As far as the theory might lead to therapeutic applications, such are not at all substantiated in the description (EPC Article 84). The claims are not clear at all (EPC Article 84). | A 61 K 31/00 |

| Place of search | Date of completion of the search | Examiner |
|---|---|---|
| The Hague | 11-09-1989 | THEUNS |

EPO Form 1504 08.78